# EUROPEAN PATENT SPECIFICATION

(11) **EP 1 609 505 B1**
(45) Date of publication and mention of the grant of the patent: **11.12.2013**
(21) Application number: 05018762.4
(22) Date of filing: 28.02.2000
(51) Int. Cl.: A61P 25/00, A61K 31/4192

(54) **Use of fluorinated triazoles for treating affective or attention disorders**
Verwendung von fluorierten Triazolen zur Behandlung von affektiven Störungen oder Aufmerksamkeitsstörungen
Utilisation de triazoles fluorés pour le traitement des troubles affectifs ou de l'attention

(30) Priority: 01.03.1999 US 259910; 01.03.1999 US 259911
(43) Date of publication of application: 28.12.2005
(62) Divisional of application: 00912515.4
(73) Proprietor: Novartis AG, 4056 Basel (CH); Novartis Pharma GmbH, 1230 Wien (AT)
(72) Inventor: Schmutz, Markus, 4124 Schönenbuch (CH)
(74) Representative: Selby, David Sean

(56) References cited:
- EP-A- 0 199 262
- GB-A- 1 511 195
- DATABASE PHARMAPROJECTS [Online] 1999, "Rufinamide" XP002152066 Database accession no. 2514
- "Novartis highlights clinical projects to turn around fortunes at its pharma division" PROMT, 4 October 1999 (1999-10-04), XP002152067

## Description

The present invention relates to a new pharmaceutical use of fluorinated triazoles.

More particularly the present invention relates to a new pharmaceutical use of compounds of the formula 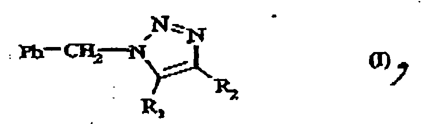 wherein Ph is o-fluorinated phenyl, which may be additionally substituted by 1 or 2 halogen atoms selected from the group consisting of fluorine and chlorine, R₁ is hydrogen, carbamoyl, N-(C₂-C₅)alkanoylcarbamoyl or N,N-di(C₁-C₄)alkylcarbamoyl; and R₂ is carbamoyl, N-(C₂-C₅)alkanoylcarbamoyl or N,N-di(C₁-C₄)alkylcarbamoyl.

The compounds of the formula I as well as their production process are known, e.g., from European Patent No. 199 262. This patent also discloses the use of the compounds of the formula I for the treatment of convulsions of different origin, e.g. of epilepsy.

In accordance with the present invention, it has now surprisingly been found, that the compounds of the formula I are useful in the treatment of affective disorders, including bipolar mood disorders, or attention disorders.

The activity of the compounds of the formula I in the treatment of affective or attention disorders is evidenced, for example, in the following tests suitable for detecting drugs having potential behavioural disinhibitory and/or sociotropic effects, which are thought to be relevant for recovery from social withdrawal, a cardinal feature of depression and related psychiatric conditions.

### a) The half enclosed platform test

This test is basically as described in Psychopharmacology, 1986, 89:31-37.

Groups of 12 male OF-1 mice are given vehicle or the substance 1 hour before being tested on the platform. The apparatus consists of a transparent platform perforated with 25 equally-spaced 1 cm holes. The platform is divided into equal halves by a 15 cm high, semi-rectangular wall enclosing one half of the platform, the other half having open edges. The whole platform rests on four 15 cm high legs. A line down the middle runs from the edge of one wall to the edge of the opposite wall. The experiment consists of placing a mouse on the midline and recording its behaviour for 5 minutes as it explores the platform. In particular, the mean frequencies and durations of the behavioural elements are recorded and statistical comparisons are determined using the Kruskal-Wellis "H" test followed by paired comparisons between control and treatment groups using the Mann-Whitney U-test. Probabilities (p=/<0.05) quoted are 2-tailed.

At doses of about 0.3 to about 10 mg/kg p.o., the compounds of the formula I significantly increase exploratory behaviour, such as stretched attend posture, head raising and forward locomotion, in the open half of the platform, while decreasing the frequency of stationary elements, such as sitting still and inactivity, in the endosed half of the platform.

### b) The intruder mouse test

This test is basically as described in Triangle, 1982, 21:95-105, and J. Clin. Psychiatry. 1994, 55:9 (suppl. B) 4-7.

A grouped "intruder" mouse is given the substance or the vehicle 1 hour before being confronted for 6 minutes with an untreated isolated, aggressive mouse (resident) in a neutral cage. Each group consists of 8 mice. The social encounter is videotaped and an observer records occurrence and duration of over 60 behavioural elements covering the non-social and social forms of the animals' behavioural repertoire. Frequency, duration and, when required, the sequence of the elements are recorded. The median frequencies and durations of the behavioural elements are recorded for each category and statistical comparisons are determined using the Kruskal-Wallis "H" test followed by paired comparisons between control and treatment groups using the Mann-Whitney "U" test. Probabilities (p=/<0.05) quoted are 2-tailed.

At doses of about 1 to about 10 mg/kg p.o., the compounds of the formula I significantly increase non-social behaviour and social investigation in the treated intruder mouse, while reducing defensive ambivalence, arrested flight and escape.

In view of their behavioural disinhibitory (= anxiolytic-/antidepressant- like) and sociotropic activity, the compounds of the formula I are useful in the treatment of affective disorders, including bipolar disorders, e.g. manic-depressive psychoses, extreme psychotic states, e.g. mania, schizophrenia, and excessive mood swings where behavioural stabilization is desired. In addition, the compounds are indicated in ADHD (attention deficit hyperactivity disorders) and other attention disorders, e.g. autism, anxiety states, generalized anxiety and agoraphobia, as well as those behavioural states characterized by social withdrawal, e.g. negative symptoms.

In a preferred group of compounds of the formula I used according to the invention, Ph is o-fluorophenyl, 2,5-4ifluorophenyl, 2,6-difluorophenyl or 2-chloro-6-fluorophonyl, R₁ is hydrogen or carbamoyl and R₂ is carbamoyl. The compound 1-(2,6-difluorophenyl)methyl-1H-1,2,3-triazole-4-carboxamide is particularly preferred.

For the above-mentioned indications the appropriate dosage will of course vary depending upon, for example, the compound employed, the host, the mode of administration and the nature and severity of the condition being treated. However, in general, satisfactory results in animals are indicated to be obtained at a daily dosage of from about 1 to about 50 mg/kg animal body weight. In larger mammals, for example humans, an indicated daily dosage is in the range of from about 50 to about 3500 mg of a compound used according to the invention conveniently administered, for example, in divided doses up to four times a day.

The compounds of the formula I may be administered in any usual manner, e.g. orally, for example in the form of tablets or capsules, or parenterally, for example in the form of injection solutions or suspensions.

Such compositions may be manufactured in conventional manner. Unit dosage forms may contain for example from about 10 mg to about 1500 mg of the compound of the formula I.

For example, tablets each containing 50 mg, or film-coated tablets each containing 100 mg, of 1-(2,6-difluorophenyl)methyl-1H-1,2,3-triazole-4-carboxamide, may be prepared as described in Examples 15 and 16 of EP 199262.

The invention provides the use of a compound of the formula I for the preparation of a medicament for the treatment of affective or attention disorders.

## Claims

1. A compound of the formula I for use in the treatment of a disorder selected from affective disorders and attention disorders 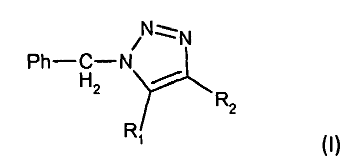 wherein Ph is o-fluorinated phenyl, which may be additionally substituted by 1 or 2 halogen atoms selected from the group consisting of fluorine and chlorine; R₁ is hydrogen, carbamoyl, N-(C₂-C₅)alkanoylcarbamoyl or N,N-di(C₁-C₄)alkylcarbamoyl; and R₂ is carbamoyl, N-(C₂-C₅)alkanoylcarbamoyl or N,N-di(C₁-C₄)alkylcarbamoyl.

2. The compound for use according to claim 1, **characterized in that** the compound of formula I is 1-(2,6-difluorophenyl)methyl-1H-1,2,3-triazole-4-carboxamide.

3. The compound for use according to claim 1 or claim 2, for treating a disorder selected from affective disorders.

4. The compound for use according to claim 1 or claim 2, for treating a disorder selected from bipolar disorders and bipolar mood disorders.

5. The compound for use according to claim 1 or claim 2, for treating a disorder selected from extreme psychotic states; manic-depressive psychoses; and mania.

6. The compound for use according to claim 1 or claim 2, for treating schizophrenia; excessive mood swings, where behavioural stabilization is desired; or social withdrawal as a feature of depression and related psychiatric conditions.

7. The compound for use according to claim 1 or claim 2, wherein the compound exerts antidepressant-like activity.

8. The compound for use according to claim 1 or claim 2, for the treatment of social withdrawal as a feature of psychiatric conditions related to depression.

9. The compound for use according to claim 1 or claim 2, for the treatment of attention disorders.

10. The compound for use according to claim 1 or claim 2, for the treatment of attention deficit hyperactivity disorders (ADHD) and other attention disorders.

11. The compound for use according to claim 1 or claim 2, for the treatment of attention disorders other than attention deficit hyperactivity disorders (ADHD).

12. The compound for use according to claim 1 or claim 2, for the treatment of autism; anxiety states; generalized anxiety; agoraphobia; behavioural states **characterized by** social withdrawal, optionally wherein the behavioural states **characterized by** social withdrawal are negative symptoms.

13. The compound for use according to claim 1 or claim 2, **characterized in that** the daily dosage is from 1 to 50 mg of the compound of the formula I /kg body weight.

14. The compound for use according to claim 1 or claim 2, **characterized in that** for a human the daily dosage is from 50 to 3500 mg of the compound of the formula I, optionally further **characterized in that** the daily dosage is administered in up to four divided doses.

15. The compound for use according to claim 1 or claim 2, **characterized in that** the compound is administerable orally, optionally further **characterized in that** the compound is in the form of a tablet or a capsule.

16. The compound for use according to claim 1 or claim 2, **characterized in that** the compound is administerable parenterally, optionally further **characterized in that** the compound is in the form of an injection solution or an injection suspension.

17. The compound for use according to claim 1 or claim 2, **characterized in that** the compound is in the form of a unit dosage containing from about 10 to about 1500 mg of the compound of the formula I; optionally wherein the dosage is a tablet containing 50 mg of the compound of the formula I mentioned in claim 2; or optionally wherein the dosage is a film-coated tablet containing 100 mg of the compound of the formula I mentioned in claim 2.

## Patentansprüche

1. Verbindung der Formel I zur Verwendung in der Behandlung einer Störung, die ausgewählt wird aus affektiven Störungen und Aufmerksamkeitsstörungen 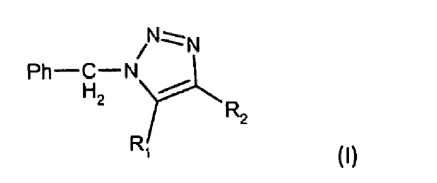 wobei Ph ein o-fluoriertes Phenyl ist, das zusätzlich substituiert werden kann durch 1 oder 2 Halogenatome, die aus der Gruppe ausgewählt werden, die Fluor und Chlor umfasst; wobei R₁ Wasserstoff, Carbamoyl, N-(C₂-C₅)Alkanoylcarbamoyl oder N,N-Di(C₁-C₄)Alkylcarbamoyl ist; und wobei R₂ Carbamoyl, N-(C₂-C₅)Alkanoylcarbamoyl oder N,N-Di(C₁-C₄)Alkylcarbamoyl ist.

2. Verbindung zur Verwendung nach Anspruch 1, **dadurch gekennzeichnet, dass** die Verbindung nach Formel I 1-(2,6-Difluorphenyl)methyl-1H-1,2,4-Triazol-4-Carboxamid ist.

3. Verbindung zur Verwendung nach Anspruch 1 oder Anspruch 2 zur Behandlung einer Störung, die aus affektiven Störungen ausgewählt wird.

4. Verbindung zur Verwendung nach Anspruch 1 oder Anspruch 2 zur Behandlung einer Störung, die aus bipolaren Störungen und bipolaren Gemütszustandsstörungen ausgewählt wird.

5. Verbindung zur Verwendung nach Anspruch 1 oder Anspruch 2 zur Behandlung einer Störung, die ausgewählt wird aus extremen psychotischen Zuständen, manisch-depressiven Psychosen und Manie.

6. Verbindung zur Verwendung nach Anspruch 1 oder Anspruch 2 zur Behandlung von Schizophrenie, übermäßigen Stimmungsschwankungen, für welche eine Verhaltensstabilisierung gewünscht wird, oder sozialem Rückzug als ein Merkmal der Depression sowie verwandter psychiatrischer Zustände.

7. Verbindung zur Verwendung nach Anspruch 1 oder Anspruch 2, wobei die Verbindung eine Aktivität ähnlich einem Antidepressivum bewirkt.

8. Verbindung zur Verwendung nach Anspruch 1 oder Anspruch 2 zur Behandlung von sozialem Rückzug als ein Merkmal psychiatrischer Zustände, die in Verbindung mit Depression stehen.

9. Verbindung zur Verwendung nach Anspruch 1 oder Anspruch 2 zur Behandlung von Aufmerksamkeitsstörungen.

10. Verbindung zur Verwendung nach Anspruch 1 oder Anspruch 2 zur Behandlung von Aufmerksamkeitsdefizit-/Hyperaktivitätsstörungen (ADHS) und anderen Aufmerksamkeitsstörungen.

11. Verbindung zur Verwendung nach Anspruch 1 oder Anspruch 2 zur Behandlung von anderen Aufmerksamkeitsstörungen als Aufmerksamkeitsdefizit-/Hyperaktivitätsstörungen (ADHS).

12. Verbindung zur Verwendung nach Anspruch 1 oder Anspruch 2 zur Behandlung von Autismus, Angstzuständen, generalisierter Angststörung, Agoraphobie, Verhaltensstörungen, die durch sozialen Rückzug gekennzeichnet sind, wobei die durch sozialen Rückzug gekennzeichneten Verhaltensstörungen optional negative Symptome sind.

13. Verbindung zur Verwendung nach Anspruch 1 oder Anspruch 2, **dadurch gekennzeichnet, dass** die Tagesdosis pro kg Körpergewicht zwischen 1 und 50 mg der Verbindung der Formel I liegt.

14. Verbindung zur Verwendung nach Anspruch 1 oder Anspruch 2, **dadurch gekennzeichnet, dass** die Tagesdosis für einen Menschen zwischen 50 und 3500 mg der Verbindung der Formel I liegt, wobei die Tagesdosis ferner optional in bis zu vier einzelnen Dosierungen verabreicht wird.

15. Verbindung zur Verwendung nach Anspruch 1 oder Anspruch 2, **dadurch gekennzeichnet, dass** die Verbindung oral verabreicht wird, wobei die Verbindung optional in Form einer Tablette oder einer Kapsel vorgesehen ist.

16. Verbindung zur Verwendung nach Anspruch 1 oder Anspruch 2, **dadurch gekennzeichnet, dass** die Verbindung parenteral verabreichbar ist, wobei die Verbindung optional in Form einer Injektionslösung oder einer Injektionssuspension vorgesehen ist.

17. Verbindung zur Verwendung nach Anspruch 1 oder Anspruch 2, **dadurch gekennzeichnet, dass** die Verbindung in Form einer Dosierungseinheit vorliegt, die zwischen etwa 10 und etwa 1500 mg der Verbindung der Formel I enthält; wobei es sich bei der Dosierung optional um eine Tablette handelt, die 50 mg der Verbindung der Formel I aus Anspruch 2 enthält; oder wobei es sich bei der Dosierung optional um eine Filmtablette handelt, die 100 mg der Verbindung der Formel I aus Anspruch 2 enthält.

## Revendications

1. Composé de la formule I destiné à être utilisé dans le traitement d'un trouble choisi parmi des troubles affectifs et des troubles de l'attention 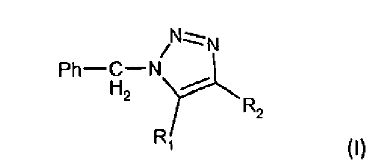 où Ph est un groupe phényle o-fluoré, qui peut être en outre substitué par 1 ou 2 atomes d'halogène choisis dans le groupe constitué par le fluor et le chlore ; R₁ est hydrogène, carbamoyle, N-(C₂-C₅)alkanoylcarbamoyle ou N,N-di(C₁-C₄)alkylcarbamoyle ; et R ₂ est carbamoyle, N-(C₂-C₅)alkanoylcarbamoyle ou N,N-di(C₁-C₄)alkylcarbamoyle.

2. Composé destiné à être utilisé selon la revendication 1, **caractérisé en ce que** le composé de la formule I est 1-(2,6-difluorophényl)méthyl-1H-1,2,3-triazole-4-carboxamide.

3. Composé destiné à être utilisé selon la revendication 1 ou 2, pour le traitement d'un trouble choisi parmi les troubles affectifs.

4. Composé destiné à être utilisé selon la revendication 1 ou 2, pour le traitement d'un trouble choisi parmi les troubles bipolaires et les troubles bipolaires de l'humeur.

5. Composé destiné à être utilisé selon la revendication 1 ou 2, pour le traitement d'un trouble choisi entre des états psychotiques extrêmes ; des psychoses maniaco-dépressives ; et la manie.

6. Composé destiné à être utilisé selon la revendication 1 ou 2, pour le traitement de la schizophrénie ; des sautes d'humeur excessives, où la stabilisation comportementale est souhaitée ; ou le retrait social comme caractéristique d'une dépression et de troubles psychiatriques associés.

7. Composé destiné à être utilisé selon la revendication 1 ou 2, dans lequel le composé exerce une activité similaire à un antidépresseur.

8. Composé destiné à être utilisé selon la revendication 1 ou 2, pour le traitement du retrait social comme caractéristique de troubles psychiatriques associés à la dépression.

9. Composé destiné à être utilisé selon la revendication 1 ou 2, pour le traitement des troubles de l'attention.

10. Composé destiné à être utilisé selon la revendication 1 ou 2, pour le traitement de troubles déficitaires de l'attention avec hyperactivité (TDAH) et d'autres troubles de l'attention.

11. Composé destiné à être utilisé selon la revendication 1 ou 2, pour le traitement de troubles de l'attention autres que les troubles déficitaires de l'attention avec hyperactivité (TDAH).

12. Composé destiné à être utilisé selon la revendication 1 ou 2, pour le traitement de l'autisme ; des états d'anxiété ; de l'anxiété généralisée ; de l'agoraphobie ; des états comportementaux **caractérisés par** le retrait social, éventuellement lorsque les états comportementaux **caractérisés par** le retrait social sont des symptômes négatifs.

13. Composé destiné à être utilisé selon la revendication 1 ou 2, **caractérisé en ce que** la dose quotidienne est de 1 à 50 mg du composé de la formule I / kg de poids corporel.

14. Composé destiné à être utilisé selon la revendication 1 ou 2, **caractérisé en ce que** pour un être humain, la dose quotidienne est de 50 à 3 500 mg du composé de la formule I, éventuellement **caractérisé en outre en ce que** la dose quotidienne est administrée en un maximum de quatre doses divisées.

15. Composé destiné à être utilisé selon la revendication 1 ou 2, **caractérisé en ce que** le composé est administrable par voie orale, éventuellement en outre **caractérisé en ce que** le composé est sous la forme d'un comprimé ou d'une capsule.

16. Composé destiné à être utilisé selon la revendication 1 ou 2, **caractérisé en ce que** le composé est administrable par voie parentérale, éventuellement **caractérisé en outre en ce que** le composé est sous la forme d'une solution injectable ou d'une suspension injectable.

17. Composé destiné à être utilisé selon la revendication 1 ou 2, **caractérisé en ce que** le composé est sous la forme d'une dose unitaire contenant environ 10 à environ 1500 mg du composé de la formule I ; éventuellement dans lequel le dosage est un comprimé contenant 50 mg du composé de la formule I mentionné à la revendication 2 ; ou éventuellement dans lequel le dosage est un comprimé pelliculé contenant 100 mg du composé de la formule I indiqué à la revendication 2.
